# EUROPÄISCHE PATENTSCHRIFT

(11) **EP 2 556 045 B1**
(45) Veröffentlichungstag und Bekanntmachung des Hinweises auf die Patenterteilung: **13.11.2013**
(21) Anmeldenummer: 11711947.9
(22) Anmeldetag: 05.04.2011
(51) Int. Cl.: C07C 209/16, C07C 209/26, C07C 211/08

(54) **VERFAHREN ZUR HERSTELLUNG VON UNSYMMETRISCHEN SEKUNDÄREN TERT.-BUTYLAMINEN IN DER GASPHASE**
METHOD FOR PRODUCING UNSYMMETRICAL SECONDARY TERT-BUTYL AMINES IN THE GAS PHASE
PROCÉDÉ DE PRODUCTION DE TERT.-BUTYLAMINES SECONDAIRES DISSYMÉTRIQUES EN PHASE GAZEUSE

(30) Priorität: 07.04.2010 EP 10159244
(43) Veröffentlichungstag der Anmeldung: 13.02.2013
(73) Patentinhaber: BASF SE, 67056 Ludwigshafen (DE)
(72) Erfinder: MÜLLER, Christoph, 68165 Mannheim (DE); WIGBERS, Christof Wilhelm, 68167 Mannheim (DE); MELDER, Johann-Peter, 67459 Böhl-Iggelheim (DE); HADERLEIN, Gerd, 67269 Grünstadt (DE); GUTFRUCHT, Norbert, 67466 Lambrecht (DE)
(86) Internationale Anmeldenummer: PCT/EP2011/055286
(87) Internationale Veröffentlichungsnummer: WO 2011/124577

(56) Entgegenhaltungen:
- WO-A1-2005/110969
- J.C. STOWELL AND S.J. PADEGIMAS: "Preparation of Sterically Hindered Secondary Amines", SYNTHESIS, Nr. 2, 1974, Seiten 127-128, XP002643797, DOI: 10.1055/s-1974-23260

## Beschreibung

Die vorliegende Anmeldung betrifft ein Verfahren zur Herstellung von unsymmetrischen sekundären tert.-Butylaminen durch kontinuierliche Aminierung in der Gasphase, wobei tert.-Butylamin in Gegenwart von einem Alkohol oder Aldehyd und Wasserstoff an Hydrierkatalysatoren umgesetzt wird.

Sekundäre Amine stellen wichtige, technisch genutzte Stoffe dar. Sie dienen beispielsweise als Polymerisations- und Härtungskatalysatoren für die Herstellung von Kunststoffformkörpern auf der Basis von Epoxiden und Polyurethanen, als Korrosionsinhibitoren und als Ausgangsstoffe für Flockungsmittel und Detergenzien. Weiterhin werden sekundäre Amine als Zwischenprodukte im Pflanzenschutz verwendet.

Unsymmetrische Amine mit tert.-Butyl- und Alkylresten sind als Ausgangsstoffe für die Herstellung von Vulkanisationsbeschleunigern für Kautschuk in WO-A 2009/084538 beschrieben und von besonderem Interesse.

Sekundäre Amine sind durch Alkylierung von primären Aminen mit Alkylhalogeniden, durch Acetylierung von primären Aminen und anschließende Reduktion der Carbonylgruppe mit Lithiumaluminiumhydrid und durch reduktive, insbesondere hydrierende Aminierung von Aldehyden mit primären Aminen zugänglich.

Dies gilt prinzipiell auch für tert.-Butylgruppen enthaltende unsymmetrische sekundäre Amine:
J. C. Bottaro et al. beschreibt in Journal of Organic Chemistry, 1991, 56, Seiten 1305 bis 1307, dass Ethyl-tert.-butylamin durch Umsetzung von tert.-Butylamin mit Ethylbromid im Molverhältnis 3 : 1 mit 85 %iger Ausbeute herstellbar ist. Nachteilig an diesem Verfahren ist, dass der anfallende Bromwasserstoff nach Neutralisation zu Salzanfall führt, dass überschüssiges tert.-Butylamin aus wirtschaftlichen Gründen abgetrennt und zurückgeführt werden muss und dass Korrosionsprobleme auftreten.
M. Newcomb et al. beschreibt hingegen in Journal of the American Chemical Society, 1990, 112, Seiten 5186 bis 5193, das tert.-Butylamin mit Acetanhydrid zu acetylieren (Ausbeute 40 %) und das erhaltene N-tert.-Butylacetamid mit Lithiumaluminiumhydrid zu Ethyl-tert.-butylamin zu reduzieren. Das Verfahren ist jedoch zweistufig, ermöglicht nur niedrige Ausbeuten und ist durch den Anfall von sauerstoffhaltigen Aluminium-verbindungen belastet.
Yu. Smirnow et al. beschreibt in Zhurnal Organicheskoi Khimii (1992), 28 (3), Seiten 461 bis 467, dass Ethyl-tert.-butylamin auch durch elektrochemische reduktive Aminierung von Acetaldehyd mit tert.-Butylamin an Bleikathoden in 60 %iger Ausbeute herstellbar ist. Nachteilig sind vor allem die niedrigen Ausbeuten.

Die Aminierung von Alkoholen mit primären Aminen und Wasserstoff in Gegenwart von Hydrierkatalysatoren zu entsprechenden unsymmetrischen sekundären Aminen ist ebenfalls bekannt. So wird bereits in EP-A 233.317 beschrieben, dass sekundäre und tertiäre Amine durch Umsetzung von primären oder sekundären Alkoholen, die 1 bis 5 Kohlenstoffatome enthalten, mit primären oder sekundären Aminen in der Gasphase in Gegenwart von Wasserstoff und Kupfer und Chromoxide (42 Gew.-% CuO, 38 Gew.-% Cr₂O₃,20 Gew.-% Al₂O₃) enthaltenden Katalysatoren hergestellt werden können. Ein Verfahren zur Herstellung von unsymmetrischen tert-Butylaminen in hohen Ausbeuten wird nicht beschrieben. Das einzige Ausführungsbeispiel zur Synthese von sekundären Aminen ist die Umsetzung von n-Butanol mit Ethylamin im Molverhältnis 3 zu 1 bei 174 - 180°C zu N-Ethyl-n-butylamin. Der n-Butanol-Umsatz beträgt dabei 52,9%, die N-Ethyl-n-butyl-Selektivität 81,6%. Nachteilig ist auch der hohe Destillationsaufwand für die Aufarbeitung des Hydrieraustrags, der zu 69,2 Gew.-% aus unumgesetztem n-Butanol, zu 8,1 Gew.-% aus unumgesetztem Ethylamin und nur zu 16,7 Gew.-% aus dem Zielprodukt N-Ethyl-n-butylamin besteht. Ein weiterer Nachteil besteht in der Verwendung von Katalysatoren, die aufgrund ihres Chromgehaltes im Hinblick auf ihre Umweltbelastung problematisch sind.

DE-A 198 59 776 beschreibt, dass man die Aminierung in der Gasphase bei Temperaturen von 80 bis 300°C, bevorzugt bei 120 bis 270°C, besonders bevorzugt bei 160 bis 250°C durchführt. Die Drücke betragen 1 bis 400 bar, bevorzugt 1 bis 100 bar, besonders bevorzugt 1 bis 50 bar. Die Katalysatorbelastung liegt im Bereich von 0,01 bis 2, bevorzugt 0,05 bis 0,5 kg Alkohol pro Liter Katalysator (Schüttvolumen) und Stunde. Pro Mol Alkohol oder Aldehyd arbeitet man mit stöchiometrischen, unter- oder überstöchiometrischen Mengen an primären oder sekundären Aminen, bevorzugt mit ca. stöchiometrischen Mengen. Als Katalysatoren werden Kupfer und sauerstoffhaltige Verbindungen des Titans als Formkörper eingesetzt, die unter Zusatz von metallischem Kupfer hergestellt werden. Tert.-Butylamin ist zwar in der allgemeinen Formel III der DE-A 198 59 776 enthalten, als bevorzugtes Aminierungsmittel wird es aber gerade in der Reihe der C₄-Alkylamine explizit ausgelassen.

Aus WO-A 2005/110969 geht hervor, dass sich Alkohole, insbesondere aber Aldehyde in der Gasphase mit primären oder sekundären Aminen in Gegenwart von Wasserstoff und Kupfer enthaltenden Katalysatoren isotherm zu sekundären oder tertiären Aminen umsetzen lassen. Die Aminierung wird bei 80 bis 300°C, bevorzugt 150 bis 250°C, besonders bevorzugt 170 bis 230°C und Absolutdrücken von 1 bis 300 bar, bevorzugt 1 bis 50 bar, besonders bevorzugt 1 bis 30 bar durchgeführt. Die Katalysator-Belastung liegt im Bereich von 0,1 bis 2,0, bevorzugt 0,1 bis 1,0, besonders bevorzugt 0,2 bis 0,6 kg Alkohol pro Liter Katalysator und Stunde. Die katalytisch aktive Masse des Katalysator-Vorläufers enthält vor der Reduktion mit Wasserstoff Aluminiumoxid, Zirkoniumdioxid, Titandioxid und/oder Siliziumdioxid. Die Aminkomponente wird bevorzugt in der 0,9 bis 100- fachen molaren Menge, insbesondere der 1 bis 10-fachen molaren Menge, jeweils bezogen auf den eingesetzten Alkohol oder Aldehyd eingesetzt. Ausführungsbeispiele zur Herstellung von sekundären Aminen I, ausgehend von Alkoholen oder Aldehyden und tert.-Butylamin sind in den zwölf Beispielen nicht enthalten.

In Advanced Synthesis & Catalysis, 2002, 344, Seite 1041, Kapitel 3.1, erster Absatz, ist beschrieben, dass bei der Umsetzung eines Amins mit einer Carbonylverbindung Ausbeuten und Selektivitäten in hohem Maße von der sterischen Hinderung der Ausgangsverbindungen abhängen. Diese sterische Hinderung spielt laut Advanced Synthesis & Catalysis auch eine Rolle im Bereich der Aminfunktion (Kapitel 3.1, dritter Absatz). So entsteht bei der reduktiven Aminierung von Aceton mit 2,4,6-Trimethyl-anilin das sekundäre Amin nur in 36 %iger, mit Anilin dagegen mit 98 %iger Ausbeute (Kapitel 3.1, dritter Absatz und Schema 10). Aus Advanced Synthesis & Catalysts ist daher für den Fachmann die Lehre abzuleiten, dass bei der Aminierung von Alkoholen bzw. Aldehyden mit tert.-Butylamin mit niedrigen Ausbeuten und Selektivitäten für das unsymmetrische, sekundäre tert.-Butylamin der Formel I zu rechnen ist.

In Synthesis, Nr. 2, 1974, Seiten 127-128 wird von J.C. Stowell und S.J. Padegimas in einem Veröffentlichung mit dem Titel "Preparation of Sterically Hindered Secondary Amines" ein Verfahren zur Herstellung von unsymmetrischen sekundären tert.-Butylaminen durch Umsetzung von tert-Butylamin mit einem Keton in Anwesenheit einer katalytischen Menge an Titantetrachlorid, gefolgt durch Hydrierung über Platinoxid bei 4 Atomsphären und Umgebeungstemperatur offenbar. Der Einsatz des Ketons erfolgt hierbei in Gegenwart einer Lewis-Sauren Katalysators (TiCl4), damit das entsprechende Imin gebildet werden kann. Der Einsatz Lewis-saurer Katalysatoren ist jedoch beim Einsatz von Aldehyden oder Alkoholen neben dem tert.Butylamin gemäß dem erfinderischen Verfahren nicht notwendig, so dass das erfindungsgemäße Verfahren ein vereinfachtes und kostengünstigeres Verfahren darstellt.

Es bestand daher die Aufgabe, ein kontinuierliches Verfahren zur Aminierung von Alkoholen oder Aldehyden mit tert.-Butylamin und Wasserstoff in Gegenwart von Hydrierkatalysatoren bereitzustellen, das es ermöglicht die an sich sterisch stark gehinderten unsymmetrischen sekundären tert-Butylamine der Formel I mit guten Ausbeuten und hoher Selektivität zu erhalten ohne Einsatz von umweltbelastenden Katalysatoren.

Eine weitere Aufgabe des Verfahrens ist es, eine einfache und ergiebige Aufarbeitung des Verfahrensproduktes zu gewährleisten

Diese Aufgabe wird gelöst durch ein Verfahren zur Herstellung von unsymmetrischen sekundären tert-Butylaminen der Formel I 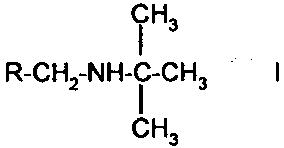 wobei R ausgewählt ist aus der Gruppe von Wasserstoff, linearen oder verzweigten aliphatischen Resten mit einem bis 15 Kohlenstoffatomen, cycloaliphatischen Resten mit 5 bis 10 Kohlenstoffatomen, Aralkylresten oder Phenylresten, die in o-, m- und/oder p-Stellung durch aliphatische Reste mit einem bis 4 Kohlenstoffatomen substituiert sein können, durch kontinuierliche Aminierung von Alkoholen der Formel II oder Aldehyden der Formel III mit tert-Butylamin und Wasserstoff in der Gasphase in Gegenwart von Hydrierkatalysatoren, umfassend folgende Schritte:
(i) Bereitstellung eines mit dem Hydrierkatalysator befüllten Reaktors
(ii) Erhitzen des Reaktors auf Temperaturen im Bereich von 60 bis 240°C und Anlegen eines Druckes im Bereich von 1 bis 100 bar,
(iii) Kontinuierliche Zugabe von Wasserstoff, tert-Butylamin und einem Alkohol der Formel II

   R - CH₂OH II

   oder einem Aldehyd der Formel III

   R-CHO III

   in den Reaktor nach Schritt (ii), wobei das molare Verhältnis von Alkohol der Formel II oder Aldehyd der Formel III zu tert-Butylamin im Bereich von 0,5 zu 1 bis 1,4 zu 1 liegt und R sowohl für den Alkohol der Formel II als auch für den Aldehyd der Formel III die gleiche Bedeutung hat wie R unter Formel I
(iv) Abkühlen und Entspannen des Reaktors und Entnahme des aus Schritt (iii) gewonnenen Hydrieraustrages .

Die erfindungsgemäße Umsetzung mit einem Alkohol der Formel II lässt sich durch die folgende Formelgleichung beschreiben:

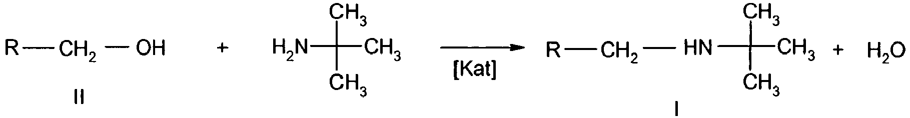

Die Aminierung wird kontinuierlich in der Gasphase durchgeführt.

Tert.-Butylamin mit einem Siedepunkt von 44°C bei 1013 mbar, ein Alkohol der Formel I

R-CH₂-OH II

oder ein Aldehyd der Formel III

R-CHO III

und gegebenenfalls Lösungsmittel werden dem Hydrierreaktor gasförmig zugeführt, wobei sowohl bei dem unsymmetrischen sekundären tert.-Butylamin der Formel I als auch dem Alkohol der Formel II und dem Aldehyd der Formel III, R ausgewählt ist aus der Gruppe von Wasserstoff, linearem oder verzweigtem aliphatischen Resten mit einem bis 15 Kohlenstoffatomen, cycloaliphatischen Resten mit 5 bis 10 Kohlenstoffatomen, Aralkylresten oder Phenylresten, die in o-, m- und/oder p-Stellung durch aliphatische Reste mit einem bis 4 Kohlenstoffatomen substituiert sein können. Bevorzugt sind die Aralkyl- oder Phenylreste mit aliphatischen Resten ausgewählt aus der Gruppe von Methyl-, Ethyl- n-Propyl-, i-Propyl-, n-Butyl-, i-Butyl, sek.-Butyl- und oder tert.-Butylgruppen substituiert.

Bevorzugte primäre Alkohole der Formel II sind ausgewählt aus der Gruppe von Methanol, Ethanol, n-Propanol, n-Butanol, 2-Methyl-1-propanol, Pivalinalkohol, n-Pentanol, n-Hexanol, 2-Ethylhexanol, 2-Methyl-1-pentanol, 3-Methyl-1-pentanol, 4-Methyl-1-pentanol, n-Octanol, n-Decanol, n-Undecanol, n-Dodecanol, 2-Phenyl-ethanol, 2-Cyclopentylethanol, 2-Cyclohexylethanol, 2-Cycloheptylethanol, Methyl-phenylethanol, Benzylalkohol, Methylbenzylalkohol oder Gemische dieser Alkohole geeignet.

Besonders bevorzugte primäre Alkohole der Formel II sind ausgewählt aus der Gruppe von Methanol, Ethanol, n-Propanol, n-Butanol, 2-Methyl-1-propanol, n-Pentanol, oder Gemische dieser Verbindungen.

Ganz besonders bevorzugt sind primäre Alkohole der Formel II ausgewählt aus der Gruppe Methanol, Ethanol, n-Propanol, n-Butanol, 2-Methyl-1-propanol oder Gemische dieser Alkohole. Insbesondere ganz besonders bevorzugt ist der primäre Alkohol der Formel II Ethanol oder n-Butanol.

Anstelle der primären Alkohole II können auch die durch Dehydrierung der Alkohole II entstehenden Aldehyde III als Ausgangsverbindungen in der Gasphase eingesetzt werden, wobei in diesem Fall mindestens 1 Äquivalent Wasserstoff während der Hydrierung im Reaktor vorhanden sein muss.

Bevorzugt ist jedoch die Verwendung von primären Alkoholen der Formel II. Die Aminierung des erfindungsgemäßen Verfahrens wird bei Temperaturen im Bereich von 60 bis 240°C, bevorzugt im Bereich von 80 bis 230°C durchgeführt. Die Aminierung der Alkohole der Formel II erfolgt bei Temperaturen von 150 bis 240°C, bevorzugt 170 bis 230°C, besonders bevorzugt 180 bis 220°C. Wird die Aminierung mit den Aldehyden der Formel III als Ausgangsverbindungen durchgeführt, so werden Temperaturen von 60 bis 200°C, bevorzugt 80 bis 170°C, besonders bevorzugt 100 bis 150°C eingehalten.

Nach der Stöchiometrie der Aminierung ist ausgehend von Alkoholen kein Wasserstoff erforderlich. Es empfiehlt sich jedoch, Wasserstoff zuzuführen, bevorzugt in einer Menge von 150 bis 250 NL, besonders bevorzugt in Mengen von 180 bis 220 NL Wasserstoff pro Liter Katalysator und Stunde. Bei Verwendung von Aldehyden der Formel III muss mindestens ein Mol Wasserstoff pro Mol Aldehyd III vorhanden sein.

Der Gesamtdruck im Reaktor bei der jeweiligen Temperatur setzt sich aus den Partialdrücken der Einsatzstoffe und der Reaktionsprodukte, also Wasserstoff, tert.-Butylamin, Alkohol der Formel II oder Aldehyd der Formel III, unsymmetrisches sekundäres tert.-Butylamin I, Wasser und gegebenenfalls einem mitverwendeten Lösungsmittel zusammen. Durch Aufpressen von Wasserstoff wird der Druck auf den gewünschten Reaktionsdruck erhöht. Um den Verbrauch an Wasserstoff auszugleichen, wird der Gesamtdruck während der Reaktionszeit durch Nachpressen von Wasserstoff konstant gehalten.

Der Gesamtdruck beträgt 1 bis 100 bar, bevorzugt 1 bis 50 bar, besonders bevorzugt 1 bis 25 bar, insbesondere bevorzugt 1 bis 20 bar.

Das Molverhältnis von Alkohol der Formel II oder Aldehyd der Formel III zu tert.-Butyl-amin liegt bevorzugt im Bereich von 1,4 zu 1 bis 0,5 zu 1, besonders bevorzugt im Bereich von 1,0 zu 1 bis 0,5 zu 1, ganz besonders bevorzugt im Bereich von 0,8 zu 1 bis 0,5 zu 1. Durch die Wahl dieser Molverüältnisse wird ein hoher Umsatz der Alkohole der Formel II oder der Aldehyde der Formel III erreicht. Gleichzeitig wird durch die Wahl dieser Molverhältnisse eine hohe Selektivität für das sekundäre Amin der Formel I bewirkt.

Es kann vorteilhaft sein, das erfindungsgemäße Verfahren in Gegenwart eines unter den Reaktionsbedingungen inerten Lösungsmittels durchzuführen. Diese inerten Lösungsmittel sind dabei ausgewählt aus der Gruppe von N-Methylpyrrolidon oder Ether wie Tetrahydrofuran, Dioxan, Ethylenglykoldimethylether.

Bevorzugt ist jedoch, in Abwesenheit eines Lösungsmittels zu arbeiten.

Die Katalysator-Belastung liegt im allgemeinen im Bereich von 0,05 bis 0,5 bevorzugt 0,1 bis 0,4, besonders bevorzugt 0,2 bis 0,3 kg Alkohol der Formel II oder Aldehyd der Formel III pro Liter Katalysator (Schüttvolumen) und Stunde.

Als Katalysatoren für das erfindungsgemäße Verfahren können alle dem Fachmann bekannten Hydrierkatalysatoren verwendet werden, wie sie zum Beispiel in Houben-Weyl, Methoden der organischen Chemie, 4. Auflage, Band 11/1, Seite 602, und Handbook of heterogeneous catalysis, 2. Auflage, Band 7, Seite 3548, Wiley VCH beschrieben sind. Als Katalysatoren kommen demnach die Metalle und/oder die Sauerstoffverbindungen der Metalle Nickel, Kobalt, Ruthenium, Rhodium, Palladium, Platin und Kupfer oder Gemische dieser Metalle und/oder der Sauerstoffverbindungen dieser Metalle in Frage. Kobalt, Kupfer und Nickel sind auch als Raney-Katalysatoren geeignet.

Bevorzugt für das erfindungsgemäße Verfahren unter der Verwendung von Alkoholen der Formel II sind Kupferoxide enthaltende Katalysatoren, wobei das Kupferoxid auf oxidische Träger aufgebracht ist. Die Menge an Kupferoxid, gerechnet als CuO, beträgt dabei 1 bis 70 Gew.-%, bevorzugt 2 bis 65 Gew.-%, besonders bevorzugt 3 bis 60 Gew.-%, bezogen auf die Gesamtmasse des oxidischen Katalysator-Vorläufers. Dieser Katalysator-Vorläufer wird entweder vor der Hydrierung oder in der Anfangsphase der Hydrierung in Gegenwart von Alkoholen II oder Aldehyden III zu elementarem Kupfer hydriert. Als Katalysator-Träger sind z. B. Aluminiumoxid, Siliziumdioxid, Titandioxid, Zirkondioxid und/oder Aktivkohle geeignet. Unter katalytisch aktiver Masse ist in diesem Zusammenhang die Summe aus Sauerstoff enthaltenden Kupferverbindungen und oxidischen Trägem zu verstehen. Besonders bevorzugt ist ein Katalysator-Vorläufer, der zu 1 bis 70 Gew.-% Kupferoxid und der fehlende Rest zu 100 % aus Aluminiumoxid besteht.

Die katalytisch aktive Masse der im erfindungsgemäßen Verfahren eingesetzten Katalysatoren kann weiterhin ein oder mehrere Elemente der Oxidationsstufe 0 oder deren anorganische oder organische Verbindungen, ausgewählt aus den Gruppen I A bis VI A und I B bis VII B und VIII des Periodensystems der Elemente enthalten.

Die Herstellung von geträgerten Kupfer-Katalysatoren ist in den Anmeldungen WO 2005/110969 und DE 19859776 detailliert beschrieben. Der Inhalt dieser Anmeldungen wird in vollem Umfang in die vorliegende Anmeldung einbezogen.

Bevorzugt wird für das erfindungsgemäße Verfahren eine Katalysatorbelastung in einem Bereich von 0,1 bis 0,3kg tert.-Butylamin pro LI Katalysator pro Stunde eingestellt. In diesem Bereich ist sowohl die Selektivität für das sekundäre Amin der Formel alls auch der Umsatz des eingesetzten tert-Butylamins günstig. Bei höheren Katalysatorbelastungen ist die Umsetzung des eingesetzten tert-Butylamin gering und bei niedrigeren Katalysatorbelastungen wird in verstärktem Maß Nebenprodukt (tertiäres Amin der Formel IV) gebildet womit die Selektivität für das Produkt (sekundäres Amin der Formel I) zurückgeht. Besonders bevorzugt wird eine Katalysatorbelastung von 0,15 bis 0,25kg tert.-Butylamin pro L Katalysator pro Stunde eingestellt. Besonders bevorzugt werden diese Katalysatorbelastungen in Kombination mit der Verwendung eines CuO-haltigen Katalysators eingestellt, insbesondere mit einem solchen, dessen Katalysator-Vorläufer zu 1 bis 70 Gew.-% aus Kupferoxid und zu dem zu 100 % verbleibenden Anteil aus Aluminiumoxid besteht

Als Reaktoren dienen bevorzugt Rohrreaktoren, wobei das erfindungsgemäße Verfahren bevorzugt in Kreisgasfahrweise durchgeführt wird. Unter Kreisgasfahrweise ist dabei zu verstehen, dass nicht umgesetzter Wasserstoff nicht aus dem Verfahren ausgeschleust, sondern zusammen mit unter den Reaktionsbedingungen der Hydrieraustrags-Kondensation gasförmigen Verbindungen in den Hydrierreaktor zurückgeführt wird.

Die oxidischen Katalysator-Vorläufer werden gemahlen, mit Formhilfsmitteln vermischt, zu Tabletten, Kugeln, Ringen oder Strängen verformt und entweder außerhalb oder im Reaktor mit Wasserstoff reduziert und im Reaktor fest angeordnet.

Die Edukte werden verdampft und kontinuierlich in Sumpf- oder Rieselfahrweise über den im Reaktor befindlichen Katalysator geleitet.

Es ist auch möglich, das erfindungsgemäße Verfahren in einem Wirbelbett mit in auf- und abwirbelnder Bewegung befindlichem Katalysatormaterial durchzuführen.

Der bei der Kondensation des gasförmigen Reaktionsaustrages anfallende Gasstrom, der überschüssigen Wasserstoff und gegebenenfalls Ausgangsverbindungen enthält, wird in die Hydrierung zurückgeführt (Kreisgas). Das Kreisgas kann in einer bevorzugten Ausführungsform zur Verdampfung der Edukte verwendet werden und enthält gleichzeitig auch den Reaktionspartner Wasserstoff für das erfindungsgemäße Verfahren.

Das Kreisgas enthält bevorzugt mindestens 10, besonders bevorzugt 50 bis 100, ganz besonders bevorzugt 80 bis 100 Volumen-% Wasserstoff.

Die Kreisgasmenge liegt bei Betriebsdruck bevorzugt im Bereich von 40 bis 1000 m³ pro m³ Katalysator und Stunde, insbesondere von 100 bis 700 m³ pro m³ Katalysator und Stunde. Die Ausgangsstoffe werden nach Verdampfung im Kreisgas dem Reaktor gasförmig zugeführt. Es ist aber auch möglich, die Edukte zu verdampfen und das Kreisgas gasförmig zuzumischen.

Die aus dem Kreisstrom ausgeschleuste Abgasmenge liegt in einem Bereich von 5 bis 800 Normkubikmetern pro Stunde, insbesondere 20 bis 300 Normkubikmetern pro Stunde.

Geeignete Reaktoren für eine Kreisgasfahrweise sind in Ullmanns Encylopedia of Industrial Chemistry, 5. Auflage Band B 4, Seiten 199 - 238, "Fixed-Bed Reactors" beschrieben. Abbildung 2 in DE 198 59 776 zeigt eine kontinuierlich betreibbare Gasphasen-Druckapparatur, in der die Edukte in das Kreisgas geführt werden.

Ganz besonders bevorzugt wird die Aminierung in einem Rohrbündelreaktor oder in einer Monostranganlage durchgeführt. Bei einer Monostranganlage besteht der Rohrreaktor aus einer Hintereinanderschaltung mehrerer, z.B. zweier oder dreier einzelner Rohrreaktoren.

Vorteilhaft für das erfindungsgemäße Verfahren ist es, wenn der Hydrieraustrag nach dem Schritt (iv) des erfindungsgemäßen Verfahrens noch entsprechend aufgearbeitet wird. Der nach Kühlung und Entspannung anfallende flüssige Hydrieraustrag enthält neben dem Zielprodukt, dem unsymmetrischen, sekundären tert.-Butylamin der Formel I, als Nebenprodukte geringe Mengen an tertiären tert.-Butylaminen der Formel IV 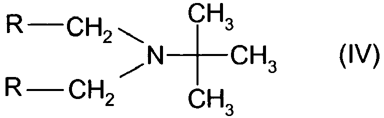 weiterhin überschüssiges tert.-Butylamin und gegebenenfalls geringe Mengen an Alkoholen der Formel II. Unter geringen Mengen sind dabei jeweils weniger als 5 Gew.-%, bevorzugt weniger als 3 Gew.-%, besonders bevorzugt weniger als 1 Gew.-% der jeweils genannten Verbindungen zu verstehen.

Bei der Aminierung entstehen etwa 5 bis 20 Gew.-% Wasser, bezogen auf die Menge des katalysatorfreien Hydrieraustrags. Die unsymmetrischen, sekundären tert.-Butyl-amine der Formel I bilden mit Wasser Azeotrope. Daher lassen sich destillativ nur Gemische aus dem Hydrieraustrag abtrennen, die Reaktionswasser und Amin I enthalten.

In EP-B 1312599 und EP-B 1312600 ist die Auftrennung von aminhaltigen Gemischen beschrieben, die ein oder mehrere Amine, Wasser, Leichtsieder und Schwersieder enthalten. Die Auftrennung erfolgt durch
(i) die destillative Abtrennung von Leichtsiedern von dem aminhaltigen Gemisch,
(ii) gegebenenfalls destillative Abtrennung von Schwersiedern von dem aminhaltigen Gemisch,
(iii) Extraktion des aminhaltigen Gemischs mit Natronlauge unter Gewinnung einer wässrigen, Natronlauge enthaltenden ersten Phase und einer wässrig-organischen, Amin enthaltenden zweiten Phase,
(iv) Destillation der wässrig-organischen zweiten Phase unter Gewinnung von Amin/Wasser-Azeotrop und von im wesentlichen wasserfreien Amin, und Rückführung des Amin/Wasser-Azeatrops in den Extraktionsschritt

Das im Wesentlichen wasserfreie Amin muss destillativ weiter gereinigt werden. In einem Ausführungsbeispiel werden die Teilschritte der Aufarbeitung an einem Hydrieraustrag demonstriert, der durch reduktive Aminierung von 1,5-Pentandiol mit Ammoniak unter Bildung von Piperidin erhalten wurde.

Bevorzugt erfolgt für das erfindungsgemäße Verfahren ebenfalls eine Aufarbeitung des Hydrieraustrags, die sowohl eine Destillation als auch die Brechung eines Amin/Wasser-Azeotrops mit einer wässrigen Alkali- und/oder Erdalkalihydroxidlösung umfasst. Sowohl die Destillation als auch die Brechung des Amin/Wasser-Azeotrops können diskontinuierlich oder kontinuierlich durchgeführt werden.

Im Gegensatz zum Stand der Technik erfolgt für das erfindungsgemäße Verfahren als erstes die Brechung des unsymmetrischen, sekundären tert.-Butylamins der Formel I / Wasser-Azeotrops durch Behandlung des Hydrieraustrags mit wässriger, Alkali-und/oder Erdalkalihydroxidlösung. Die Alkali- und/oder Erdalkalihydroxidkonzentration in der wässrigen Lösung kann 1 bis 75 Gew.-%, bevorzugt 25 bis 50 Gew.-%, betragen. Bevorzugte wässrige Alkali- und/oder Erdalkalihydroxidlösungen sind ausgewählt aus der Gruppe von Natronlauge, Kalilauge, Magnesiumhydroxid, Calciumhydroxid. Bevorzugt ist Natronlauge. Besonders bevorzugt ist eine 50 Gew.-%ige Natronlauge.

Nach Extraktion des Hydrieraustrags mit der wässrigen Alkali- und/oder Erdalkali-hydroxidlösung wird diese durch Phasentrennung abgetrennt. Der Restwassergehalt der organischen Phase kann zum Beispiel durch Karl-Fischer-Titration ermittelt werden. Die für die Wasserabtrennung benötigte Menge an Alkali- und/oder Erdalkali-hydroxidlösung lässt sich durch wenige Vorversuche bestimmen.

Die für die Extraktion mit Alkali- und/oder Erdalkalihydroxidlösung eingesetzte Extraktionsvorrichtung kann ein- oder mehrstufig ausgebildet sein, beispielsweise ein einzelner Mixer-Settler-Extraktor. Mehrstufige Extraktionen sind beispielsweise Extraktionskolonnen oder Extraktionskaskaden. Als Extraktionskolonnen eignen sich beispielsweise Füllkörper-, Siebboden-, Kaskaden-, Pulsations-, Rotations- und Zentrifugalkolonnen. Eine Extraktionskaskade sind beispielsweise mehrere hintereinander geschaltete Mixer-Settler-Extraktoren, die auch platzsparend als Turmextraktor oder Kastenextraktor ausgeführt sein können. Ist der Extraktor mehrstufig, so ist bevorzugt eine Gegenstrom-Extraktionskolonne mit im allgemeinen 1 bis 25, bevorzugt 4 bis 10 theoretischen Trennstufen bevorzugt. Diese wird im allgemeinen bei einem Druck betrieben, bei dem alle Komponenten des Extraktionsgemisches unterhalb ihres Siedepunktes vorliegen, und sich ferner eine Viskosität der beiden Phasen einstellt, bei der eine Dispergierung der beiden Phasen problemlos möglich ist. Die Temperatur beträgt im Allgemeinen 5 bis 200°C, bevorzugt 20 bis 70°C, besonders bevorzugt 40 bis 50°C. Nach Phasentrennung wird die wässrige Alkali- und/oder Erdalkalihydroxidlösung enthaltende Phase aus dem Verfahren ausgeschleust.

Falls die abgetrennte wässrige Alkali- und/oder Erdalkalihydroxidlösung wesentliche Mengen an unsymmetrischen, sekundärem tert.-Butylamin der Formel I, Alkohol der Formel II und/oder Aldehyd der Formel III und/oder tert.-Butylamin enthält, so können diese Verbindungen durch Extraktion mit organischen Lösungsmitteln zurück gewonnen werden. Wesentliche Mengen liegen dann vor, wenn die Summe der obigen Verbindungen mehr als 10 Gew.-%, bevorzugt mehr als 5 Gew.-%, besonders bevorzugt mehr als 2 Gew.-%, bezogen auf den wasser- und katalysatorfreien Hydrieraustrag beträgt.

Als organische Lösungsmittel kommen dabei zum Beispiel aliphatische, cycloaliphatische oder aromatische Kohlenwasserstoffe in Frage, die mit wässriger Alkaliund/oder Erdalkalihydroxidlösung eine Mischungslücke besitzen. Beispiele für derartige Kohlenwasserstoffe sind n-Hexan, n-Octan, Cyclohexan, Toluol und Ethylbenzol oder Gemische dieser Verbindungen.

Die wässrige Alkali- und/oder Erdalkalihydroxidlösungsphase wird von der Kohlenwasserstoffphase durch Phasentrennung abgetrennt. Aus der Kohlenwasserstoffphase wird der Kohlenwasserstoff destillativ entfernt. Das zurück gewonnene unsymmetrische, sekundäre tert.-Butylamin der Formel I, der Alkohol der Formel II und/oder der Aldehyd der Formel III und/oder tert.-Butylamin kann mit der Hauptmenge an rohem unsymmetrischen, sekundären tert.-Butylamin der Formel I, das aus der ersten organischen Phase nach Phasenabtrennung durch Extraktion gewonnen wurde vereinigt und destillativ aufgereinigt werden.

Es ist weiterhin möglich, das Azeotrop aus unsymmetrischem, sekundärem tert.-Butyl-amin der Formel I und Wasser durch Zugabe von Kohlenwasserstoffen zum Hydrieraustrag, Abdestillieren von Kohlenwasserstoff/Wasser-Heteroazeotropen aus dem Hydrieraustrag, Abtrennen der Wasser- von der Kohlenwasserstoffphase und Rückführung der Kohlenwasserstoffphase in die Destillation zu brechen.

Eine weitere Möglichkeit besteht darin, zunächst das Azeotrop aus unsymmetrischem sekundärem tert.-Butylamin der Formel I und Wasser destillativ abzutrennen und dann erst die Entwässerung durch Natronlauge-Behandlung oder Destillation mit Kohlenwasserstoffen durchzuführen.

Schließlich lässt sich die Wasserentfemung durch Alkali- und/oder Erdalkalihydroxidlösungsbehandlung mit der Wasserentfernung durch Destillation mit Kohlenwasserstoffen verknüpfen. Hierbei entfernt man die Hauptmenge an Wasser im Hydrieraustrag durch Alkali- und/oder Erdalkalihydroxidlösungsbehandlung, beispielsweise durch einstufige Extraktion mit Alkali- und/oder Erdalkalihydroxidlösung, trennt die Phasen, vereinigt die abgetrennte Kohlenwasserstoffphase mit dem katalysatorfreien Hydrieraustrag und entfernt das noch vorhandene Wasser oder einen Teil davon durch Azeotropdestillation.

In einem besonders bevorzugten Verfahren wird das Wasser vor der destillativen Aufarbeitung des Hydrieraustrags nicht vollständig abgetrennt. Bevorzugt ist es, wenn der Wassergehalt des Hydrieraustrags unter 5 Gew.-%, bevorzugt unter 3 Gew.-%, besonders bevorzugt unter 0,9 Gew.-% beträgt. Liegt nur wenig Restwasser vor, so wird bei der Destillation auch nur wenig unsymmetrisches, sekundäres tert.-Butylamin der Formel I als Azeotrop mit Wasser ausgeschleust. Kleine Mengen an Azeotrop, beispielsweise solche, die weniger als ein Mol-% unsymmetrisches, sekundäres tert.-Butylamin der Formel I, bezogen auf eingesetztes tert.-Butylamin enthalten, können gegebenenfalls verworfen werden. Es ist aber auch möglich, das Azeotrop in die Alkali- und/oder Erdalkalihydroxidlösungsextraktion zurückzuführen. Vorteilhaft ist, mit einer einstufigen Alkali- und/oder Erdalkalihydroxidlösungsbehandlung des Hydrieraustrags auszukommen. Dabei muss keine Feinabstimmung der Alkali- und/oder Erdalkalihydroxidlösungsmenge erfolgen, um auch die letzten Reste an Wasser zu entfernen.

Der wasserfreie oder nur noch weniger als 5, bevorzugt weniger als 3, besonders bevorzugt weniger als 1 Gew.-% Wasser enthaltende Hydrieraustrag kann durch fraktionierende Destillation weiter aufgereinigt werden. Die Destillation kann, abhängig von den zu destilliererenden Mengen, kontinuierlich oder diskontinuierlich durchgeführt werden. Dabei gehen in ersten Mischfraktionen, falls vorhanden, nicht umgesetztes tert.-Butylamin, nicht umgesetzter Alkohol der Formel II und aus den Aldehyden der Formel III entstandene Alkohole über Kopf ab. Dann folgt das unsymmetrische, sekundäre tert.-Butylamin der Formel I, das ebenfalls über Kopf abdestilliert wird. Im Sumpf verbleiben, falls vorhanden, tertiäre Amine der Formel IV und Hochsieder. Nach GC-Analyse weniger als 97 Flächen-%, besonders bevorzugt weniger als 98 Flächen-%, besonders bevorzugt weniger als 99 Flächen-% sekundäres Amin enthaltende Fraktionen können in die Destillation zurückgeführt werden.

Für die fraktionierende Destillation kommen übliche Apparaturen in Betracht, wie sie beispielsweise in Kirk-Othmer, Encyclopedia of Chemical Technology, 3. Edition, Volume 7, John Wiley and Sons, New York,1979, Seiten 870 bis 881 beschrieben sind. Bevorzugt sind dabei Siebbodenkolonnen, Glockenbodenkolonnen, Packungskolonnen oder Füllkörperkolonnen.

Durch fraktionierende Destillation werden Reinheiten der unsymmetrischen, sekundären tert.-Butylamine der Formel I von mehr als 98 FI.-%, insbesondere mehr als 99 FI.-%, besonders bevorzugt von mehr als 99,5 FI.-%, insbesondere mehr als 99,9 erzielt (GC-Analyse).

Für die kontinuierliche Aufarbeitung nach der Offenbarung EP-B 1312599 und EP-B 1312600, Fig. 1 und 2 werden drei bis vier maßgeschneiderte Destillations-kolonnen und eine Extraktionsvorrichtung benötigt.

Bei der erfindungsgemäßen diskontinuierlichen Aufarbeitung werden dagegen nur eine Destillationskolonne und eine Extraktionsvorrichtung verwendet.

### Beispiele

### Beispiel 1

### Aminierung von Ethanol mit tert.-Butylamin in der Gasphase

Der Versuch wurde in einem ölbeheizten Reaktor aus Glas (1 m Länge, 40 mm Durchmesser) bei Umgebungsdruck durchgeführt. Der Reaktor wurde in Rieselfahrweise betrieben. In den unteren Teil des Reaktors wurden 300 ml V2A-Netzringe (5 mm Durchmesser), darüber 200 ml (180 g) mit Wasserstoff bei 180 bis 200°C reduzierter und anschließend mit Sauerstoff passivierter Kupfer-Katalysator (3 x 3 mm Tabletten) gefüllt. Der Kupfer-Katalysator bestand vor der Reduktion und Passivierung zu 55 Gew.-% aus Kupferoxid(CuO) und zu 45 Gew.-% aus Aluminiumoxid Der obere Teil des Reaktors wurde mit 500 ml V2A-Netzringen beschickt. Der Reaktor wurde durch einen Ölkreislauf beheizt. Wasserstoff und Gemische aus tert.-Butylamin und Alkohol der Formel II wurden kontinuierlich in den Reaktor gepumpt. Der Hydrieraustrag wurde gekühlt, entspannt und gaschromatographisch analysiert (DB 1-Säule, Temperaturprogramm 60 - 280°C, 10°C pro Minute). Die Angaben zu Selektivitäten und Umsätzen beziehen sich auf GC-Flächenprozente.

In Beispiel 1 wurden pro Stunde 40 g tert.-Butylamin (0,55 mol), 20 g Ethanol (0,44 mol) und 40 NL Wasserstoff bei 210°C über den Kupfer-Katalysator gefahren. Das Molverhältnis von tert.-Butylamin zu Alkohol betrug dabei 1 : 0.8, die Katalysator-Belastung 0.2 kg tert.-Butylamin. pro Liter Katalysator und Stunde.

In Tabelle 1 sind die Reaktionsbedingungen und die Ergebnisse der gaschromatographischen Analyse zusammengestellt.

Das erfindungsgemäße Beispiel 1 zeigt, dass die Selektivität des Zielprodukts Ethyl-tert.-butylamin 97%, die des Nebenprodukts Diethyltert.-butylamin 1 % und der tert.-Butylaminumsatz 75% beträgt.

### Beispiel 2

### a) Aminierung von Ethanol mit tert.-Butylamin in der Gasphase

Die Aminierung wurde in einem Rohrreaktor (3,5 m Länge, 40 mm Innendurchmesser) bei 220°C und 20 bar Gesamtdruck durchgeführt. Der Reaktor wurde in Rieselfahrweise betrieben. Er enthielt 1000 ml (900 g) einer frischen Charge des in Beispiel 1 verwendeten, mit Wasserstoff bei 180 bis 200°C reduzierten und anschließend mit Sauerstoff passivierten Kupfer-Katalysators (H3-82, 3x3 mm Tabletten).

Pro Stunde wurden 300 g tert.-Butylamin (4,1 mol), 96 g Ethanol (2,05 mol) und 300 NL Wasserstoff gasförmig in Rieselfahrweise über den Kupfer-Katalysator geleitet. Die Kreisgasmenge betrug 3,2 Nm³/h. Das Molverhältnis von tert.-Butylamin zu Ethanol betrug dabei 1 zu 0.5, die Katalysator-Belastung 0,3 kg tert.-Butylamin pro Liter Katalysator und Stunde.

In Tabelle 1, Beispiel 2 sind die Reaktionsbedingungen und die Ergebnisse der gaschromatographischen Analyse zusammengestellt.

Das erfindungsgemäße Beispiel 2 zeigt, dass die Selektivität des Zielprodukts Ethyl-tert.-butylamin 99%, die des Nebenprodukts Diethyltert.-butylamin 1 % und der tert.-Butylaminumsatz 59% beträgt.

### b) Entwässerung des Hydrieraustrags

Der Hydrieraustrag enthielt 12 Gew.-% Wasser. Er wurde eine Stunde lang bei Raumtemperatur mit wässriger, 50%iger Natronlauge (Volumenverhältnis Hydrieraustrag: Natronlauge = 1 : 1) gerührt. Dann wurden die Phasen getrennt. Der Wassergehalt der organischen Phase betrug nun 0,9 Gew.-%.

### c) Destillative Aufarbeitung des Hydrieraustrags

2.811 g Hydrieraustrag aus Hydrierung a), dessen Wassergehalt durch Entwässerung nach b) auf 0,9 Gew.-% verringert worden war, wurden destillativ aufgearbeitet. Verwendet wurde eine 6 L Destillationsblase mit aufgesetzter Packungskolonne (2 x 1,2 m Kolonne, Durch-messer 43 mm, Packung: Montz A 3-1000). Die Anzahl der theoretischen Trennstufen betrug 30, das Rücklaufverhältnis 10 : 1. Die Destillation erfolgte diskontinuierlich bei einem Druck von 950 mbar. Die Sumpftemperatur stieg im Verlauf der Destillation von 77°C auf 151 °C an. Bei der Destillation wurden 2.612 g Destillat (92,9 Gew.-%), 150 g Destillationsrückstand (5,3 Gew.-%) und 9 g Kühlfalleninhalt (0,3 Gew.-%), bezogen auf die in der Destillation eingesetzte Menge Hydrieraustrag, erhalten. Fraktion 1 (288 g) bestand zu 99,7 Flächen% aus tert.-Butylamin. Sie kann in die Hydrierung a) zurückgeführt werden. Die Fraktionen 2 bis 7 (809 g) stellten Gemische aus überwiegend Ethyl-tert.-butylamin, weiterhin Ethanol und Wasser dar. Diese Fraktionen können in die Destillation c), gegebenenfalls nach vorangehender Entwässerung in b), zurückgeführt werden. Die Fraktionen 8 bis 15 (1.515 g) bestanden zu 99,1 bis 99,9 Flächen% aus Ethyl-tert.-butylamin. 82 % dieser Fraktionen besaßen eine Reinheit von > 99,8 Flächen%.

Der Destillationsrückstand bestand zu 42 % aus Ethyl-tert.-butylamin und zu 35 % aus Diethyl-tert.-butylamin.

Die destillative Aufarbeitung von Beispiel 2 c) zeigt die Vorteile, die sich bei der Hydrierung a) aus einem Überschuss an tert.-Butylamin gegenüber dem Alkohol ergeben: Überschüssiges tert.-Butylamin lässt sich problemlos als Kopfprodukt von dem restlichen Hydrieraustrag abtrennen und in die Hydrierung zurückführen. Außerdem demonstriert Beispiel 2 c), dass tertiäres tert.-Butylamin IV quantitativ von dem sekundären tert.-Butylamin I abtrennbar ist und dass Reinheiten des sekundären Amins I von 99,8 Flächen% und mehr erreichbar sind.

### Vergleichsbeispiel 1

Beispiel 1 wurde mit der Änderung wiederholt, dass das Molverhältnis von tert.-Butyl-amin zu Ethanol 1 zu 2,5 betrug. Tabelle 1 zeigt, dass die Selektivität des Zielprodukts Ethyl-tert.-butylamin auf 90% sinkt und die Selektivität des Nebenprodukts Diethyltert.-butylamin auf 7% ansteigt. Der tert.-Butylaminumsatz betrug 98%.

### Beispiel 3

Beispiel 1 wurde mit der Änderung wiederholt, dass statt des Cu-Katalysators ein Cu/Ni-Katalysator (3x3 mm Tabletten; hergestellt aus Präkatalysator mit 46% CuO, 11 % NiO, 44% Al₂O₃) für die Hydrierung verwendet wurde. Tabelle 1 zeigt eine Selektivität für das Zielprodukt Ethyl-tert.-butylamin von 95% bei einer Selektivität für das Nebenprodukt Diethyltert.-butylamin von 3%. Der tert.-Butylaminumsatz betrug 75%.

### Beispiel 4

Beispiel 1 wurde mit der Änderung wiederholt, dass eine Katalysatorbelastung von nur 0,1 kg tert.-Butylamin pro Liter Katalysator und Stunde eingestellt wurde. Dazu wurden pro Stunde 20 g tert.-Butylamin (0,275 mol), 10 g Ethanol (0,22 mol) und 20 NL Wasserstoff über den Kupfer-Katalysator gefahren. Tabelle 1 zeigt eine Selektivität für das Zielprodukt Ethyl-tert.-butylamin von 96% bei einer Selektivität für das Nebenprodukt Diethyltert.-butylamin von 3%. Der tert.-Butylaminumsatz betrug 79%.

### Beispiel 5

Beispiel 1 wurde mit der Änderung wiederholt, dass die katalytische Hydrierung bei 170°C statt 210°C durchgeführt wurde. Tabelle 1 zeigt eine Selektivität für das Zielprodukt Ethyl-tert.-butylamin von 94% bei einer Selektivität für das Nebenprodukt Diethyl-tert.-butylamin von 0%. Der tert.-Butylaminumsatz betrug 40%.

### Beispiel 6

Beispiel 1 wurde mit der Änderung wiederholt, dass das Molverhältnis von tert.-Butyl-amin zu Ethanol 1 zu 1 betrug. Tabelle 1 zeigt eine Selektivität für das Zielprodukt E-thyl-tert.-butylamin von 95% bei einer Selektivität für das Nebenprodukt Diethyltert.-butylamin von 2%. Der tert.-Butylaminumsatz betrug 82%.

### Beispiel 7

Beispiel 1 wurde mit der Änderung wiederholt, dass n-Butanol statt Ethanol mit tert.-Butylamin aminiert wurde. Tabelle 1 zeigt eine Selektivität für das Zielprodukt n-Butyl-tert.-butylamin von 97% bei einer Selektivität für das Nebenprodukt Di-n-butyl-tert.-butylamin von 0%. Der tert.-Butylaminumsatz betrug 82%.

**Tabelle 1:**

| Beispiel | Eingesetzter Alkohol II | Hydriertemperatur [°C] | Molverhältnis tert.-Butylamin zu Alkohol II | Katalysator-Belastung [kg/l Kat · h] | Tert.-Butylamin-Umsatz [%] | Selektivität sek. Amin I [%] | Selektivität tert.-Amin IV |
|---|---|---|---|---|---|---|---|
| 1 | Ethanol | 210 | 1 : 0,8 | 0,2 | 75 | 97 | 1 |
| 2 | Ethanol | 220 | 1:0,5 | 0,3 | 59 | 99 | 1 |
| Vergleichsbeispiel 1 | Ethanol | 210 | 1:2,5 | 0,2 | 98 | 90 | 7 |
| 3¹⁾ | Ethanol | 210 | 1 : 0,8 | 0,2 | 75 | 95 | 3 |
| 4 | Ethanol | 210 | 1 : 0,8 | 0,1 | 79 | 96 | 3 |
| 5 | Ethanol | 170 | 1 : 0,8 | 0,2 | 40 | 94 | 0 |
| 6 | Ethanol | 210 | 1 : 1 | 0,2 | 82 | 95 | 2 |
| 7 | n-Butanol | 210 | 1 : 0,8 | 0,2 | 82 | 97²⁾ | 0 |

| | | | | | | | |
|---|---|---|---|---|---|---|---|
| ¹⁾ Katalysator: Cu/Ni ²⁾ tert.-Butyl-butylamin | | | | | | | |

## Patentansprüche

1. Verfahren zur Herstellung von unsymmetrischen sekundären tert-Butylaminen der Formel I 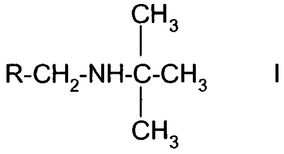 wobei R ausgewählt ist aus der Gruppe von Wasserstoff, linearen oder verzweigten aliphatischen Resten mit einem bis 15 Kohlenstoffatomen, cycloaliphatischen Resten mit 5 bis 10 Kohlenstoffatomen, Aralkylresten oder Phenylresten, die in o-, m- und/oder p-Stellung durch aliphatische Reste mit einem bis 4 Kohlenstoffatomen substituiert sein können,
durch kontinuierliche Aminierung von Alkoholen der Formel II oder Aldehyden der Formel III mit tert.-Butylamin und Wasserstoff in der Gasphase in Gegenwart von Hydrierkatalysatoren, umfassend folgende Schritte:
(i) Bereitstellung eines mit dem Hydrierkatalysator befüllten Reaktors
(ii) Erhitzen des Reaktors auf Temperaturen im Bereich von 60 bis 240°C und Anlegen eines Druckes im Bereich von 1 bis 100 bar,
(iii) Kontinuierliche Zugabe von Wasserstoff, tert.-Butylamin und einem Alkohol der Formel II
R - CH₂OH II
oder einem Aldehyd der Formel
R-CHO III III
in den Reaktor nach Schritt (ii), wobei das molare Verhältnis von Alkohol der Formel II oder Aldehyd der Formel III zu tert-Butylamin im Bereich von 0,5 zu 1 bis 1,4 zu 1 liegt und R sowohl für den Alkohol der Formel II als auch für den Aldehyd der Formel III die gleiche Bedeutung hat wie R unter Formel I
(iv) Abkühlen und Entspannen des Reaktors und Entnahme des aus Schritt (iii) gewonnenen Hydrieraustrages.

2. Verfahren nach Anspruch 1, wobei der Hydrieraustrag nach Schritt (iv) folgenden Schritten der Aufarbeitung unterzogen wird:
a) Extraktion des Hydrieraustrags aus Schritt (iv) mit einer wässrigen Alkali- und/oder Erdalkalihydroxidlösung
b) Abtrennung der aus Schritt a) gewonnenen wässrigen Phase von der organischen Phase und
c) fraktionierte Destillation der aus Schritt a) gewonnenen organischen Phase.

3. Verfahren nach Anspruch 2, wobei
α) die aus Schritt a) gewonnene wässrige Phase noch einmal mit Kohlenwasserstoffen ausgewählt aus der Gruppe von aliphatischen, cycloaliphatischen und/oder aromatischen Kohlenwasserstoffen, die mit wässriger Alkali- und/oder Erdalkalihydroxidlösung eine Mischungslücke aufweisen, versetzt werden, wenn der Gehalt der Summe der Verbindungen der Formel I, des Alkohols der Formel II oder des Aldehyds der Formel III und tert-Butylamin mehr als 2 Gew.-% des Hydrieraustrages aus Schritt (iv) beträgt,
β) anschließend die Kohlenwasserstoffphase von der wässrigen Phase abgetrennt wird,
γ) der Kohlenwasserstoff aus der Kohlenwasserstoffphase destillativ entfernt wird
δ) und der Rückstand der Destillation mit der aus Schritt b) gewonnen organischen Phase vereinigt wird.

4. Verfahren nach einem der Ansprüche 1 bis 3, wobei der Wassergehalt der aus Schritt b) gewonnenen organischen Phase bis zu 5 Gew.-% bezogen auf das Gesamtgewicht der nach Schritt b) erhaltenen organischen Phase beträgt.

5. Verfahren nach einem der Ansprüche 1 bis 4, wobei R in der Formel I, II und III für Methyl steht.

6. Verfahren nach einem der Ansprüche 2 bis 5, wobei die Alkali- und/oder Erdalkalihydroxidlösung für die Extraktion nach Schritt a) eine 1 bis 75 Gew.-%ige Natronlauge ist.

7. Verfahren nach einem der Ansprüche 1 bis 6, wobei als Hydrierkatalysator in Schritt i) Metalle und/oder Sauerstoffverbindungen der Metalle ausgewählt aus der Gruppe von Nickel, Kobalt, Ruthenium, Rhodium, Palladium, Platin und Kupfer oder Gemische dieser Metalle eingesetzt werden.

8. Verfahren nach Anspruch 7, **dadurch gekennzeichnet, dass** der oxidische Katalysatorvorläufer zu 1 bis 70 Gew.-% aus Kupferoxid und der fehlende Rest zu 100 % aus Aluminiumoxid besteht.

9. Verfahren nach einem der Ansprüche 1 bis 7, wobei ein Alkohol der Formel II eingesetzt wird.

10. Verfahren nach einem der Ansprüche 1 bis 8, wobei die zugeführte Wasserstoffmenge im Bereich von 150 bis 250 NL Wasserstoff pro Liter Katalysator und Stunde liegt.

11. Verfahren nach einem der Ansprüche 1 bis 9, wobei unter Abwesenheit eines Lösungsmittels gearbeitet wird.

12. Verfahren nach einem der Ansprüche 1 bis 10, wobei für die katalytische Hydrierung eine Katalysatorbelastung von 0,1 bis 0,3kg tert.-Butylamin pro L Hydrierkatalysator pro Stunde eingestellt wird.

## Claims

1. A process for preparing unsymmetric secondary tert-butylamines of the formula I 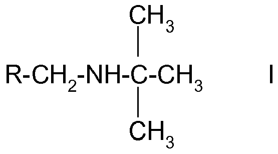 where R is selected from the group of hydrogen, linear or branched aliphatic radicals having one to 15 carbon atoms, cycloaliphatic radicals having 5 to 10 carbon atoms, aralkyl radicals or phenyl radicals which may be o-, m- and/or p-substituted by aliphatic radicals having one to 4 carbon atoms, by continuously aminating alcohols of the formula II or aldehydes of the formula III with tert-butylamine and hydrogen in the gas phase in the presence of hydrogenation catalysts, comprising the following steps:
(i) providing a reactor filled with the hydrogenation catalyst,
(ii) heating the reactor to temperatures in the range from 60 to 240°C and applying a pressure in the range from 1 to 100 bar,
(iii) continuously adding hydrogen, tert-butylamine and an alcohol of the formula II
R - CH₂OH II
or an aldehyde of the formula III
R - CHO III
to the reactor according to step (ii), where the molar ratio of alcohol of the formula II or aldehyde of the formula III to tert-butylamine is in the range from 0.5 : 1 to 1.4 : 1, and R both for the alcohol of the formula II and for the aldehyde of the formula III is as defined for R in formula I,
(iv) cooling and decompressing the reactor and withdrawing the hydrogenation output obtained from step (iii).

2. The process according to claim 1, wherein the hydrogenation output according to step (iv) is subjected to the following workup steps:
a) extracting the hydrogenation output from step (iv) with an aqueous alkali metal hydroxide and/or alkaline earth metal hydroxide solution,
b) removing the aqueous phase obtained from step a) from the organic phase and
c) fractionally distilling the organic phase obtained from step a).

3. The process according to claim 2, wherein
α) the aqueous phase obtained from step a) is admixed once more with hydrocarbons selected from the group of aliphatic, cycloaliphatic and/or aromatic hydrocarbons which have a miscibility gap with aqueous alkali metal hydroxide and/or alkaline earth metal hydroxide solution when the content of the sum of the compounds of the formula I, of the alcohol of the formula II or of the aldehyde of the formula III and tert-butylamine is more than 2% by weight of the hydrogenation output from step (iv),
β) the hydrocarbon phase is subsequently removed from the aqueous phase,
γ) the hydrocarbon is distillatively removed from the hydrocarbon phase
δ) and the residue of the distillation is combined with the organic phase obtained from step b).

4. The process according to any of claims 1 to 3, wherein the water content of the organic phase obtained from step b) is up to 5% by weight based on the total weight of the organic phase obtained after step b).

5. The process according to any of claims 1 to 4, wherein R in the formula I, II and III is methyl.

6. The process according to any of claims 2 to 5, wherein the alkali metal hydroxide and/or alkaline earth metal hydroxide solution for the extraction according to step a) is a 1 to 75% by weight sodium hydroxide solution.

7. The process according to any of claims 1 to 6, wherein the hydrogenation catalyst used in step i) comprises metals and/or oxygen compounds of the metals selected from the group of nickel, cobalt, ruthenium, rhodium, palladium, platinum and copper, or mixtures of these metals.

8. The process according to claim 7, wherein the oxidic catalyst precursor consists of copper oxide to an extent of 1 to 70% by weight and the remainder to 100% consists of aluminum oxide.

9. The process according to any of claims 1 to 7, wherein an alcohol of the formula II is used.

10. The process according to any of claims 1 to 8, wherein the amount of hydrogen supplied is in the range from 150 to 250 1 (STP) of hydrogen per liter of catalyst and hour.

11. The process according to any of claims 1 to 9, which is operated in the absence of a solvent.

12. The process according to any of claims 1 to 10, wherein a catalyst hourly space velocity of 0.1 to 0.3 kg of tert-butylamine per 1 of hydrogenation catalyst per hour is established for the catalytic hydrogenation.

## Revendications

1. Procédé pour la préparation de tert-butylamines secondaires asymétriques de formule I 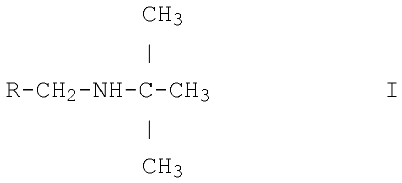 dans laquelle R est choisi dans le groupe constitué par un atome d'hydrogène, des radicaux aliphatiques linéaires ou ramifiés ayant de un à 15 atomes de carbone, des radicaux cycloaliphatiques ayant de 5 à 10 atomes de carbone, des radicaux aralkyle ou des radicaux phényle, qui peuvent être substitués en position o, m et/ou p par des radicaux aliphatiques ayant un à 4 atomes de carbone,
par amination continue d'alcools de formule II ou d'aldéhydes de formule III avec de la tert-butylamine et de l'hydrogène dans la phase gazeuse en présence de catalyseurs d'hydrogénation, comprenant les étapes suivantes :
(i) disposition d'un réacteur chargé avec le catalyseur d'hydrogénation,
(ii) chauffage du réacteur jusqu'à des températures dans la plage de 60 à 240°C et application d'une pression dans la plage de 1 à 100 bars,
(iii) introduction continue d'hydrogène, de tert-butylamine et d'un alcool de formule II
R-CH₂OH II
ou d'un aldéhyde de formule
R-CHO III III
dans le réacteur selon l'étape (ii), le rapport molaire de l'alcool de formule II ou de l'aldéhyde de formule III à la tert-butylamine se situant dans la plage de 0,5:1 à 1,4:1 et R aussi bien pour l'alcool de formule II que pour l'aldéhyde de formule III ayant la même signification que R dans la formule I
(iv) refroidissement et détente du réacteur et prélèvement de la décharge d'hydrogénation obtenue dans l'étape (iii).

2. Procédé selon la revendication 1, dans lequel après l'étape (iv) on soumet la décharge d'hydrogénation aux étapes suivantes du traitement final :
a) extraction de la décharge d'hydrogénation provenant de l'étape (iv) à l'aide d'une solution aqueuse d'hydroxyde de métal alcalin et/ou d'hydroxyde de métal alcalino-terreux
b) séparation de la phase aqueuse obtenue dans l'étape a) d'avec la phase organique et
c) distillation fractionnée de la phase organique obtenue dans l'étape a).

3. Procédé selon la revendication 2, dans lequel
α) on ajoute encore une fois à la phase aqueuse obtenue dans l'étape a) des hydrocarbures choisis dans le groupe des hydrocarbures aliphatiques, cycloaliphatiques et/ou aromatiques, qui présentent une lacune de miscibilité avec une solution aqueuse d'hydroxyde de métal alcalin et/ou de métal alcalino-terreux, lorsque la concentration de la somme des composés de formule I, de l'alcool de formule II ou de l'aldéhyde de formule III et de tert-butylamine est supérieure à 2 % en poids de la décharge d'hydrogénation provenant de l'étape (iv),
β) ensuite on sépare la phase d'hydrocarbure de la phase aqueuse,
γ) on sépare par distillation l'hydrocarbure de la phase d'hydrocarbure
δ) et on réunit le résidu de la distillation avec la phase organique obtenue dans l'étape b).

4. Procédé selon l'une quelconque des revendications 1 à 3, dans lequel la teneur en eau de la phase organique obtenue dans l'étape b) vaut jusqu'à 5 % en poids, par rapport au poids total de la phase organique obtenue après l'étape b).

5. Procédé selon l'une quelconque des revendications 1 à 4, dans lequel R dans les formules I, II et III représente le groupe méthyle.

6. Procédé selon l'une quelconque des revendications 2 à 5, dans lequel la solution d'hydroxyde de métal alcalin et/ou de métal alcalino-terreux pour l'extraction selon l'étape a) est une solution d'hydroxyde de sodium à 1 à 75 % en poids.

7. Procédé selon l'une quelconque des revendications 1 à 6, dans lequel on utilise comme catalyseur d'hydrogénation dans l'étape i) des métaux et/ou des composés oxygénés des métaux choisis dans le groupe constitué par le nickel, le cobalt, le ruthénium, le rhodium, le palladium, le platine et le cuivre ou des mélanges de ces métaux.

8. Procédé selon la revendication 7, **caractérisé en ce que** le précurseur de catalyseur de type oxyde consiste à raison de 1 à 70 % en poids en oxyde de cuivre et le reste manquant consiste à raison de 100 % en oxyde d'aluminium.

9. Procédé selon l'une quelconque des revendications 1 à 7, dans lequel on utilise un alcool de formule II.

10. Procédé selon l'une quelconque des revendications 1 à 8, dans lequel la quantité d'hydrogène introduite se situe dans la plage de 150 à 250 litres normaux d'hydrogène par litre de catalyseur et par heure.

11. Procédé selon l'une quelconque des revendications 1 à 9, dans lequel on opère en absence d'un solvant.

12. Procédé selon l'une quelconque des revendications 1 à 10, dans lequel on ajuste pour l'hydrogénation catalytique une charge de catalyseur de 0,1 à 0,3 kg de tert-butylamine par litre de catalyseur d'hydrogénation par heure.
